# EUROPÄISCHE PATENTSCHRIFT

(11) **EP 0 697 840 B1**
(45) Veröffentlichungstag und Bekanntmachung des Hinweises auf die Patenterteilung: **18.02.2004**
(21) Anmeldenummer: 94914343.2
(22) Anmeldetag: 11.05.1994
(51) Int. Cl.: A61B 18/12, A61N 5/06

(54) **VORRICHTUNG ZUR THERMISCHEN VERÖDUNG BIOLOGISCHEN GEWEBES**
DEVICE FOR THERMALLY OBLITERATING BIOLOGICAL TISSUES
DISPOSITIF PERMETTANT DE CAUTERISER DES TISSUS BIOLOGIQUES

(30) Priorität: 14.05.1993 DE 4316176; 02.02.1994 DE 4403134
(43) Veröffentlichungstag der Anmeldung: 28.02.1996
(73) Patentinhaber: CeramOptec GmbH, 53121 Bonn (DE); HÜTTINGER MEDIZINTECHNIK GmbH & CO. KG, D-79224 Umkirch (DE)
(72) Erfinder: BEUTHAN, Jürgen, D-12165 Berlin (DE); ROGGAN, André, D-13629 Berlin (DE); MÜLLER, Gerhard, D-14129 Berlin (DE)
(74) Vertreter: Altenburg, Udo, Dipl.-Phys.
(86) Internationale Anmeldenummer: PCT/DE1994/000554
(87) Internationale Veröffentlichungsnummer: WO 1994/026184

(56) Entgegenhaltungen:
- EP-A- 0 247 746
- WO-A-90/04363
- DE-A- 3 532 604
- DE-A- 4 211 526
- DE-C- 4 137 983

## Beschreibung

Die Erfindung betrifft eine Vorrichtung zur Durchführung der thermischen Verödung biologischen Gewebes mittels Laserstrahlung, die durch einen Lichtleiter in das Gewebe eingebracht wird, wobei die Laserstrahlung durch Modifikationen in Zusammenhang mit der Austrittsfläche des Lichtleiters gestreut wird.

Es ist bekannt, daß Laserstrahlung mittels optischer Wellenleiter geführt und die so transmittierte Strahlung entweder unmittelbar mittels des optischen wellenleiters transluminal bzw. transkutan in biologisches Gewebe eingebracht oder zusätzlich in ihrer Verteilungsfunktion durch weitere Maßnahmen - etwa über spezielle Katheter bzw. Endoskope - gezielt beeinflußt und die derart gezielt applizierte Strahlung zur Induzierung thermischer oder photochemischer Nekrosen genutzt werden kann. Seit Mitte der 70er Jahre ist es geübte Praxis, auf diese Weise etwa Neodym:YAG Laserstrahlung in biologisches Gewebe einzubringen und die im Gewebe auftretende Absorption dieser Strahlung zur Aufheizung der zur Faser endständigen Gewebsareale zu nutzen und damit Koagulationsnekrosen herbeizuführen.

Bedingt durch die Tatsache, daß die verwendeten Lichtleitfasern aktive Durchmesser zwischen 200 und 600 µm haben, ergibt sich an der Grenzfläche Faser/Gewebe selbst bei geringen Ausgangsleistungen des Lasers eine sehr hohe Leistungsdichte. Daher wird sehr schnell die Karbonisationsschwelle des Gewebes überschritten mit der Folge, daß die austretende Laserstrahlung zusätzlich durch das Karbonisat absorbiert wird und nicht mehr entsprechend dem gewebstypischen optischen Koeffizienten eindringen kann. Selbst dem geübtesten Operateur gelingt auf diese Weise nur die Erzeugung von Koagulationsnekrosen mit 5 bis 7 mm Durchmesser, die in der Regel mit einer Karbonisierung des das Faserende berührenden Gewebes einhergehen.

Operationstechnisch erfolgt das transkutane Einbringen der Lichtleitfaser, der sogenannten "bare-fiber", in der Regel mit Punktionssets, d.h. mit metallischen Kanülen und Trokaren. Die transluminale Anwendung erfolgt weiterführend über geeignete flexible Katheter oder Endoskope.

In US 5,169,396, die für Anspruch 1 als nachträglichsten Stand der Technik zu betrachten ist, ist eine Vorrichtung und ein Verfahren zur interstitiellen Lasertherapie beschrieben. Dabei wird eine dünne metallische Kanüle mit einem offenen Ende, in welcher eine optische Faser mit einem offenen Ende zur Führung von Laserlicht mit einer bestimmten Wellenlänge und Intensität longitudinal angepasst werden kann, in ein zu behandelndes Gewebe eingeführt. Durch die metallische Kanüle kann eine biokompatible Flüssigkeit in das zu behandelnde Gewebe geleitet werden.

Das Verfahren zeichnet sich in seinen Grundzügen dadurch aus, daß ein direkter Kontakt der Faserendfläche mit dem Gewebe dadurch unterbunden wird, daß am Ende der Punktionskanüle zunächst ein Flüssigkeitsdepot mit einer die Laserstrahlung absorbierenden biokompatiblen Flüssigkeit gesetzt wird. Somit wird primär nicht mehr die Grenzfläche Faser-Gewebe aufgeheizt, sondern die lichtabsorbierende Flüssigkeit, die dann ihrerseits über Wärmeleitung das umliegende Gewebe erwärmt.

Diese Vorgehensweise vermeidet zwar die primäre Karbonisation am Faserende, hat aber den entscheidenden Nachteil, daß der Energietransport ins Gewebe lediglich noch durch Wärmeleitung erfolgt und damit in Anbetracht der großen Wärmesenke des umgebenden Gewebes nur zu sehr begrenzten Koagulationsnekrosen führt. Weiterhin hat das Verfahren den wesentlichen Nachteil, daß die Pigmente bzw. chromophoren Gruppen der die Laserstrahlung absorbierenden Flüssigkeit bei den zur Anwendung kommenden Strahlungsleistungen und dadurch bedingten Leistungsdichten am Faserende sich ebenfalls photochemisch bzw. thermisch zersetzen und dadurch unkontrollierbare Nebenwirkungen auslösen.

DE 41 37 983 A1, die für Anspruch 4 als nachträglichsten Stand der Technik betrachtet wird offenbart eine Applikationsvorrichtung für die Behandlung biologischer Gewebe mit Laserstrahlung, wobei das dem zu behandelnden

Gewebe als Objekt zugewandte Ende eines strahlführenden Lichtwellenleiters, der als Faseroptik ausgebildet ist, in einem stimseitig für Laserstrahlung transparenten, röhrenförmigen Hüllkörper, der am objektseitigen Ende luft- und flüssigkeitsdicht verschlossen ist, angeordnet ist, wobei das Ende des Lichtwellenleiters durch eine Aufrauung an seinem distalen Ende eine ein Streuvolumen aufweisende Streuvorrichtung für Laserstrahlung bildet, die für die verwendete Laserstrahlung weitergehend transparent ist. Ferner wird offenbart, daß eine weitere Streuvorrichtung innerhalb des Hüllkörpers angeordnet ist oder den stimseitigen Abschluss des Hüllkörpers bildet.

Bei der aus DE 41 37 983 A1 bekannten Anordnung wird das Problem der Karbonisierung an der Faser-Gewebe-Grenzfläche dadurch zu lösen versucht, daß das Faserende speziell präpariert ist derart, daß die Laserstrahlung nicht mehr gerichtet prograd aus der Faser mit einer sehr geringen Austrittsapertur austritt, sondern über eine längere Strecke schrittweise radial aus der Faser herausgestreut wird. Durch weitere, die radiale Streuung noch erhöhende Maßnahmen, wie das Vorsehen eines Streudoms, wird die Verteilung der Laserleistung auf der Oberfläche des Applikators weiter vergleichmäßigt.

Durch diese Maßnahmen kann zwar das Ziel einer möglichst homogenen Einbringung der Laserstrahlung in das Gewebe weitgehend erreicht werden, die technologischen Aufwendungen zur Präparation des Faserendes und ggfs. eines hochtemperaturfesten, aber flexiblen Hüllkatheters sind jedoch erheblich. Ein weiterer Nachteil dieses Laserstreulichtapplikators mit Hüllkatheter ist, daß sich naturgemäß die dem Katheter anliegende Gewebeschicht durch die Lichtabsorption besonders schnell aufheizt und damit koaguliert, wobei sich gezeigt hat, daß das koagulierte Gewebe eine deutlich schlechtere Transmittanz für gerichtete optische Strahlung im wellenlängenbereich aufweist.

In DE 42 11 526 A1 und DE 42 37 286 A1, die beide erst nach der für dieses Patent beansprachten Priorität veröffentlicht worden sind, ist eine Vorrichtung angegeben, die mit einer aktiven Kühlung innerhalb des Hüllkatheters arbeitet, wobei die Kühlflüssigkeit gleichzeitig die Aufgabe einer zusätzlich Zerstreuung des Laserlichtes übernimmt. In einer Abwandlung ist vorgesehen, daß durch präformierte Poren im Hüllkatheter biokompatible Kühlflüssigkeit aus dem Katheter austreten kann und so eine Karbonisierung und damit auch ein Anhaften anliegenden Gewebes vermieden wird.

Bei dieser Anordnung kann zwar durch die aktive Kühlung Laserleistung von mehr als 5 W, typischerweise 10 bis 12 W, genutzt werden, jedoch wird durch die Kühlung gleichzeitig ein Teil der applizierten Energie ungenutzt wieder abgeführt. Da die biokompatible Flüssigkeit sich im Gewebe schnell verteilt, werden während einer 10 bis 20 min dauernden Behandlung zudem relativ große Mengen benötigt, die ggf. auch zu Belastungen des Patienten führen können. Schließlich sind auf diese Weise - wie auch mit den vorgenannten Lösungen - nur in etwa kugelsymmetrische Behandlungszonen realisierbar. Die Behandlung langgestreckter, aber relativ schlanker krankhafter Gewebsareale erfordert eine Mehrfachapplikation durch Repositionierung im Hüllkatheter und führt beispielsweise bei der Behandlung der benignen Prostatahyperplasie (BPH) zu sehr langen Bestrahlungszeiten pro Prostatalappen von bis zu 40 min.

Die DE-35 32 604 A1 beschreibt eine Möglichkeit zur Bestrahlung von Hohlräumen bei der Behandlung von oberflächlichen Tumoren in Hohlorganen mittels Photodynamischer Therapie. Dabei wird ein starrer hohlkugelförmiger Bestrahlungskörper beschrieben, der auch in einem Endoskop einsetzbar ist. Zur Homogenisierung der Strahlung aus einem Lichtwellenleiter wird dafür eine Streuflüssigkeit verwendet.

Die Vorrichtung der in dieser Anmeldung beschriebenen Erfindung dient hingegen der interstitiellen Anwendung von Laserstrahlung. Die Streuflüssigkeit zur Bildung eines Fluiddepots (1) steht hier in Gegensatz zu DE-35 32 604 A1 in direktem Kontakt mit dem Gewebe (2).

Der Erfindung liegt die Aufgabe zugrunde, eine Vorrichtung zur Verfügung zu stellen, die die Durchführung des Verfahrens der eingangs genannten Gattung so weiterbildet, daß eine Gewebeverödung unter effizienter Nutzung von Laserstrahlung und Streuflüssigkeit in möglichst großen und auch in nicht kugelsymmetrischen Behandlungszonen ermöglicht wird.

Diese Aufgabe wird jeweils durch eine Vorrichtung mit den Merkmalen des Anspruchs 1 sowie durch eine Vorrichtung mit den Merkmalen des Anspruchs 4 gelöst.

Das damit verbesserte Verfahren schließt den Gedanken ein, in der Umgebung der Austrittsfläche der Laserstrahlung aus einem ihrer Zuleitung dienenden Lichtwellenleiter in das zu verödende Gewebe einen den Behandlungserfordernissen in der Größe und räumlichen Gestalt möglichst genau angepaßten, während der Behandlung im wesentlichen stabilen Streubereich zu schaffen, in dem im wesentlichen keine Absorption, sondern nur eine möglichst diffuse Streuung der Laserstrahlung erfolgt. Diesen Zweck erfüllt ein Streufluid-Depot aus einer hinreichend viskosen, im Wellenlängenbereich der verwendeten Laserstrahlung im wesentlichen transparenten Flüssigkeit bzw. einem Flüssigkeitsgemisch.

Da das Fluid die Laserstrahlung nicht absorbiert und die Streuprozesse reine Phasenstreuung darstellen, heizt sich das Fluid nicht wesentlich auf, wodurch über einen großen Zeitraum - gemessen an der Gesamtbestrahlungszeit - eine Koagulation an der Strahlungsaustrittsfläche zum Gewebe unterdrückt wird.

Die Erfindung beruht auf der überraschenden Erkenntnis, daß bei Verwendung der Vorrichtung und durch Vermeidung der primären Koagulation an der Randzone Streuflüssigkeit/Gewebe die sich diffus in der Streuflüssigkeit ausbreitende Laserstrahlung um den Faktor 2 bis 3 tiefer in das Gewebe eindringen kann, und somit dort in einem deutlich größeren Volumen absorbiert wird und dieses Volumen aufheizt. Dabei tritt dann zunächst nach einer Bestrahlungszeit von mehreren Minuten in der Tiefe des Gewebes eine konzentrische Koagulationszone auf, die einen weiteren reinen Strahlungstransport in die Tiefe des Gewebes hindert, gleichzeitig aber durch erhöhte Temperatur eine volumenmäßig sehr große Wärmequelle zur Ausnutzung der Wärmeleitung zur weiteren thermischen Verödung des umliegenden Gewebes bildet.

Das das Flüssigkeitsdepot umgebende Gewebe wird erst gegen Ende der Behandlung durch Anheben der Bestrahlungsleistung des Lasers koaguliert und führt damit indirekt zu einer weiteren Aufheizung der Kühlflüssigkeit, so daß auch nach Abschalten des Lasers noch einmal durch das eingebrachte Flüssigkeitsdepot und die massive gesetzte thermische Koagulationsfront eine Wärmequelle vorhanden ist, die ihrerseits über Wärmeleistung weiterhin umliegendes Gewebe thermisch auch im subkoagulativen Bereich gemäß dem Arrhenius-Integral schädigt. Bei verfahrensgemäßem Vorgehen lassen sich so Durchmesser von Koagulationszonen von bis zu 3 cm erreichen, darüber hinaus entsteht durch die subkoagulative, hyperthermische Einflußnahme aufgrund der Wärmeleitung eine weiteren Nekrose mit einem Radius von zusätzlich ca. 5 bis 7 mm, so daß insgesamt Nekroseherde bis zu 4 cm kontrolliert gesetzt werden können.

Die in der US 5,169,396 beschriebenen Nachteile der Benutzung einer metallischen Kanüle werden in vorteilhafter Weise dadurch vermieden, daß zur Einbringung der Lichtleitfaser und der streuenden Kühlflüssigkeit in das Gewebe ein die Laserstrahlung nicht absorbierendes, biokompatibles Kanülenmaterial verwendet wird. Hierfür kommen Varianten der thermoplastischen PTFE-Familie, Polykarbonate, Varianten des HDPE und bestimmte Polyuretane in Frage.

Das operationstechnische Vorgehen wird so gewählt, daß über eine Sondiernadel zunächst der Zugang in das Zentrum des zu verödenden Gewebeareals gefunden wird, wobei dieses Vorgehen unter gleichzeitiger Röntgen- oder Ultraschallkontrolle erfolgen kann. Anschließend werden über diese Nadel je nach Größe des später zu setzenden Nekroseherdes ein oder mehrere Dilatoren geführt, wobei der letzte der Dilatoren mit der später den Laserstrahl führenden Kanüle identisch ist. Anschließend wird über diesen Kunststoffdilatator, der endständig offen ist, das biokompatible lichtstreuende Fluid in das Gewebe eingebracht.

Die verwendete Kanüle ist zweckmäßig so ausgebildet, daß sie aus im wesentlichen zwei Baugruppen besteht, deren eine die über eine Sondiernadel einzubringende Punktionskanüle selbst ist und deren zweite ein an eben dieser Punktionskanüle nach Entfernung der Sondiernadel anzubringendes fluiddichtes Y-Stück darstellt, über das einerseits das das Laserlicht streuende Fluid injiziert werden kann und andererseits der das Laserlicht führende Lichtwellenleiter vorjustiert fixiert und über einen Verschiebemechanismus definiert axial vorgeschoben werden kann.

Von besonderer Bedeutung für das Verfahren ist, daß das Fluid eine an die jeweilige Behandlungssituation angepaßte Viskosität besitzt, damit über eine hinreichend lange Bestrahlungszeit ein räumlich geeignet definiertes Flüssigkeitsdepot aufrechterhalten wird. Daher scheiden gängige biokompatible Fluide wie phyisiologische Kochsalzlösung aus, da diese eine so geringe Viskosität aufweisen, daß während der typischen Behandlungszeiten von 10 bis 20 Minuten in den für diese Therapieform insbesondere in Frage kommenden Gewebetypen wie Parenchym, Muskel, Prostata usw. mehrere 100 ml Flüssigkeit zur Aufrechterhaltung eines Depots benötigt würden. Dies läßt sich durch Wahl einer geeigneten Viskosität des Fluids vermeiden.

Das Fluid kann einfach und kostengünstig aus einer Mischung von 0,1 bis 2 Anteilen Öl, 10 bis 50 Anteilen Wasser und 50 bis 80 Anteilen Glyzerin bestehen, wobei die jeweiligen Anteils-Summen 100 betragen. In einer bevorzugten Ausführung wird eine Öl-Wasser-Glyzerin-Suspension verwendet, die folgendes Mischungsverhältnis aufweist: 80 % Glyzerin, 18 % Wasser und 2 % suspendierte Öltröpfchen.

Weiterhin kann eine Mischung aus 1 bis 30 Teilen Wasser und 70 bis 99 Teilen Methylzellulose - vorzugsweise Methylzellulose, versetzt mit wenigen Prozenten physiologischer Kochsalzlösung - verwendet werden.

Schließlich kann auch eine Mischung aus Hyalaronsäure und Intralipid eingesetzt werden.

In Abhängigkeit von der Größe und geometrischen Gestalt des Depots ist die numerische Apertur des Lichtwellenleiters zu wählen. Gute Ergebnisse werden bei numerischen Aperturen zwischen 0,2 und 0,6 erreicht. Die Verwendung einer erhöhten numerischen Apertur bietet die Möglichkeit, in einfacher Weise auch Diodenlaser bei ca. 800 nm als Energiequelle enzusetzen.

In einer vorteilhaften Ausgestaltung besteht die Flüssigkeit aus einer im wesentlichen transparenten und einer stark lichtstreuenden Komponente, und die Komponenten werden mittels einer konzentrisch doppellumigen Kanüle derart in das Gewebe injiziert, daß die transparente Komponente über das innere und die stark lichtstreuende Komponente über das äußere Lumen eingebracht werden, so daß die stark lichtstreuende Komponente die transparente Komponente umhüllt.

Die transparente Komponente kann Glyzerin und Wasser, vorzugsweise in einem Mischungsverhältnis von 80:20, und die stark streuende Komponente Öl und Wasser, vorzugsweise mit 1 bis 5 Anteilen Öl in 100 Teilen Mischung, aufweisen.

In einer konstruktiv einfachen Fortbildung dieser Ausgestaltung kann die Laserstrahlung über die gleichzeitig als Lichtwellenleiter dienende Flüssigkeitssäule der transparenten Komponente im inneren Lumen in das Gewebe eingebracht werden, wobei die Einstrahlung des Laserlichtes ggf. auch über eine instationäre Flüssigkeitssäule - also während weiterhin Flüssigkeit injiziert wird - erfolgen kann.

Zur Optimierung des Strahlungseintrages in das Fluiddepot kann nach Injektion der Flüssigkeit und Bildung des Depots der Lichtwellenleiter in distaler Richtung um einen vorbestimmten Betrag in das Depot hinein verschoben werden.

Zur Bildung langgestreckter Behandlungszonen wird das Streufluiddepot in langgestreckter, insbesondere im wesentlichen ellipsoidischer oder zylinderförmiger Gestalt, gebildet und/oder eine Lichtwellenleiteranordnung mit einer derart geformten Abstrahlfläche eingesetzt.

Diese weist in vorteilhafter Ausbildung eine Lichtleitfaser auf, deren Oberfläche in einem distalen Endabschnitt mehrere in Längsrichtung aufeinanderfolgende mattierte, vorzugsweise ringförmig ausgebildete, Bereiche aufweist, und deren Endabschnitt mit einer für die Laserstrahlung transparenten Schutzhülle versehen ist. Die mattierten Bereiche können eine zum distalen Ende der Lichtleitfaser hin zunehmende Rauhtiefe und/oder abnehmenden Durchmesser aufweisen.

Koaxial zur Lichtwellenleiteranordnung kann ein die Laserstrahlung streuendes - etwa mattiertes - Hüllrohr vorgesehen sein.

Eine Schutzhülle ist erforderlich, weil die Lichtleitfaser durch die partielle Mattierung bzw. Aufrauhung extrem bruchempfindlich wird. Sie kann in kostengünstiger weise durch eine endständig verschlossene und am mechanisch festen Teil des Lichtwellenleiters oder der Kanüle befestigten Präzisionsglas-oder Hartkunststoffkanüle gebildet sein. In einer zweckmäßigen, robusten Ausbildung des Katheters bzw. der Kanüle umschließt die Schutzhülle deren gesamtes Vorderteil und weist Öffnungen zum Austritt der die Laserstrahlung streuenden Flüssigkeit in das Gewebe auf.

Der Verlauf der Temperaturfeldausbreitung im behandelten Gewebe kann in zweckmäßiger Weise durch fortlaufende Beobachtung des Therapieareals mit einem Sonographiegerät kontrolliert wird, wobei zusätzlich Echos temperaturabhängig in dem Areal benachbarten Blutgefäßen dann auftreten, wenn hier die Temperatur einen wert von 55 °C überschreitet und damit das im Blut gelöste CO₂ intermediär ausgast unhd weitere Echos auftreten, wenn bei überschreiten einer Temperatur von 95°C in der wässerigen Komponente des Streulichtfluids intermediär Wasserdampfblasen entstehen.

Weiterhin kann der Flüssigkeit ein Röntgenkontrastmittel beigefügt und eine röntgenologische Beobachtung der Bildung und des Zustandes des Depots vorgenommen werden.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
Figuren 1a bis 1d schematische Darstellungen zur Erläuterung des Stands der Technik unter Verwendung einer einlumigen Kanüle,
Figur 2 eine schematische Darstellung zur Erläuterung einer Ausführungsform der erfindungsgemäßen Vorrichtung,
Figur 3 eine schematische Ausschnittsdarstellung (im Querschnitt) einer weiter modifizierten erfindungsgemäßen Vorrichtung,
Figur 4 eine vereinfachte, teilweise geschnittene Darstellung einer weiteren Ausführungsform,
Figur 5 eine Detaildarstellung zur in Fig. 4 gezeigten Vorrichtung,
Figur 6 eine schematische Darstellung eines Verfahrens zur Herstellung der bei den Ausführungsformen nach Fig. 4 und 5 eingesetzten Lichtwellenleiteranordnung.

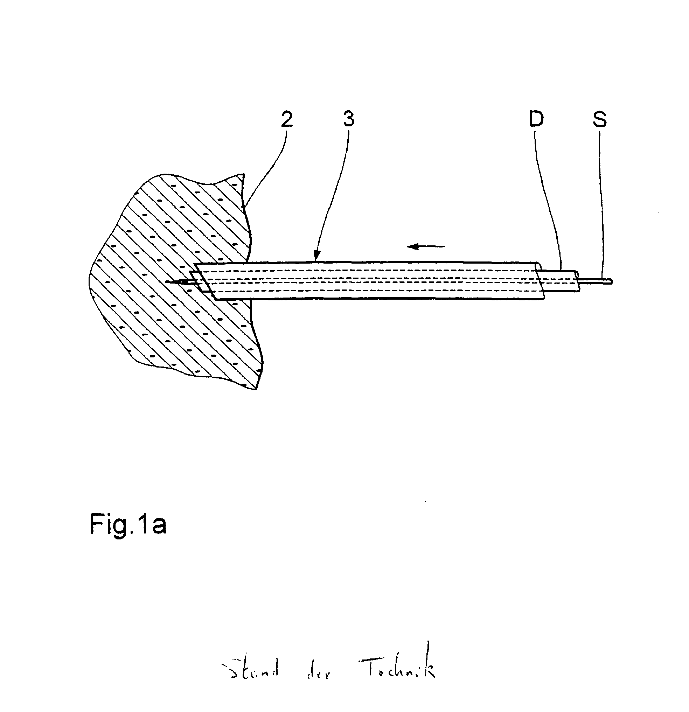

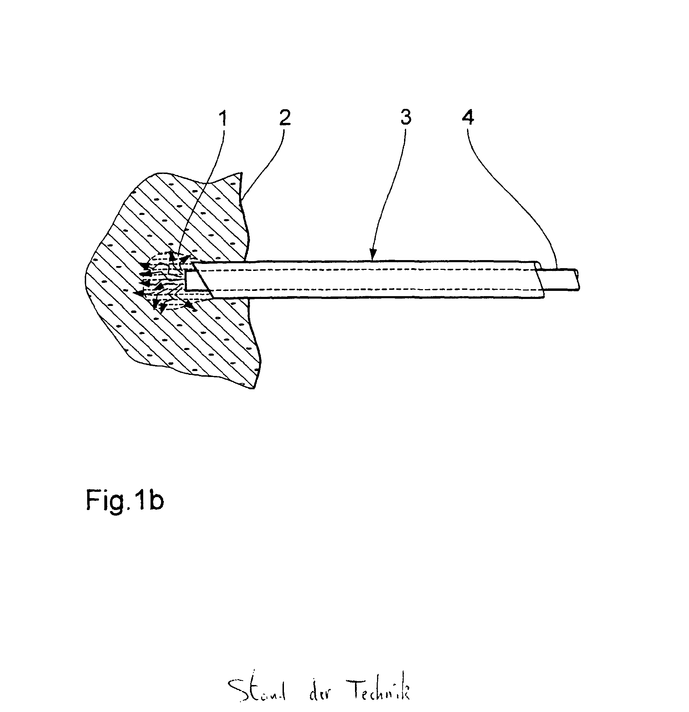

In Fig. 1a bis 1d ist schematisch dargestellt, wie die Ausbildung eines Fluiddepots 1 in einem Körpergewebeabschnitt 2 mittels einer Kanüle 3, die Streuung einer über einen Lichtleiter 4 innerhalb der Kanüle 3 eingekoppelten Laserstrahlung in dem Depot 1 und die Entstehung einer Koagulationsnekrose 5 gemäß dem Stand der Technik ablaufen.

Fig. la verdeutlicht schematisch das allgemeine Vorgehen bei Einführung einer Kanüle 3 zur Durchführung des Verfahrens zum Stand der Technik in den Körpergewebeabschnitt 2: Zunächst wird mit einem Sondierdraht bzw. einer Sondiernadel S der Zugang in das Zentrum des zu verödenden Gewebsareals gefunden. Anschließend wird über die Sondiernadel 5 ein Dilator D eingeführt, und über den Dilator D schließlich die Kanüle 3. Je nach Größe des zu bildenden Nekroseherdes können auch in mehreren Stufen mehrere Dilatoren gesetzt werden, und der letzte kann gleichzeitig die laser- und/oder flüssigkeitsführende Kanüle für die weiteren Schritte sein, wie unten genauer beschrieben wird.

In der in Fig. lb skizzierten Phase wurde zunächst über die Kanüle 3 viskose Streuflüssigkeit in das Gewebe 2 injiziert, darin das räumlich begrenzte Fluiddepot 1 gebildet und mit der Einkopplung von Laserstrahlung in das Depot begonnen. Die Photonen der Laserstrahlung werden - was durch gezackte Pfeile symbolisiert ist - in der quasi absorptionsfreien Streuflüssigkeit des Depots 1 und in das Gewebe 2 gestreut. Dadurch wird eine erste virtuelle Volumenvergrößerung bei der Energieübertragung in das zu therapierende Gewebe erreicht.

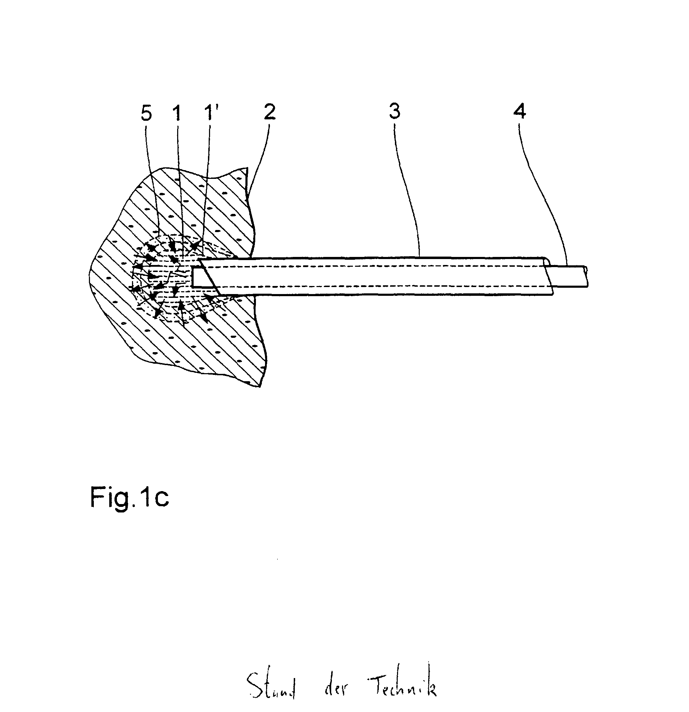

In der in Figur 1c skizzierten Phase wirkt die Streuflüssigkeit an der Phasengrenze 1' des Depots 1 zum Gewebe 2 noch kühlend, so daß eine koagulative Wirkung sich primär in der Tiefe des Gewebes ausbreitet und sich die vom Depot 1 einen Abstand aufweisende Koagulationsschicht 5 bildet. Dies bedeutet eine weitere virtuelle Vergrößerung des aktiven Applikationsvolumens beim Energieübertrag. Gleichzeitig tritt durch Wärmeleitung eine Erwärmung des quasi absorptionsfreien Streuflüssigkeitsdepots 1 ein - es gibt also einen Energieaustausch im Grenzschichtbereich, der in der Figur durch gerade Pfeile symbolisiert ist.

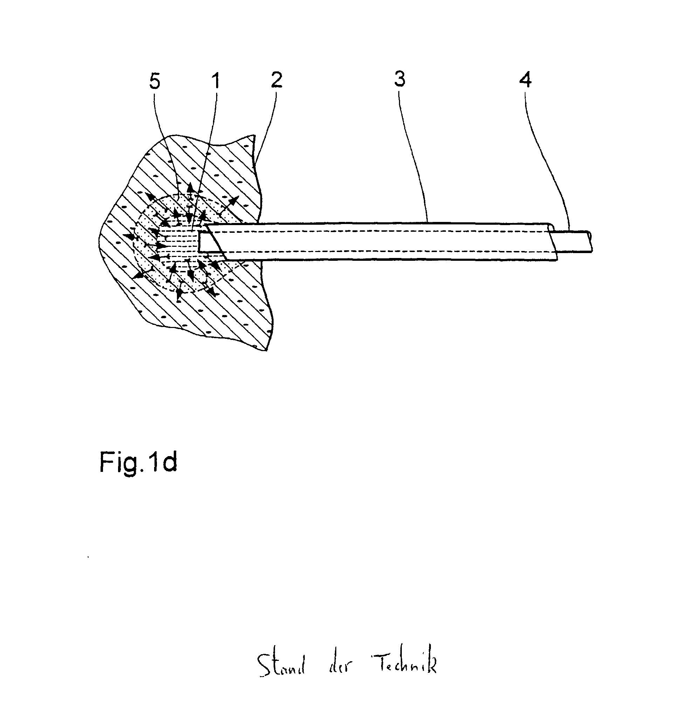

In der in Figur 1d skizzierten Phase ist die Laserstrahlung abgeschaltet. Das aufgeheizte Gesamtvolumen der interaktiven Teilvolumina bewirkt eine optimale Ausdehnung der Koagulationsnekrose 5 nach innen, d.h. zum Depot 1 hin, wie nach außen, d.h. in die Tiefe des Gewebes 2 (durch sich zum Ende hin verdickende Pfeile verdeutlicht).

Eine Kontrolle dieser thermischen Verödung von Gewebevolumina erfolgt über das Ausnutzen von zwei überraschend gefundenen Phänomenen:
1. Bei vitalen biologischen Geweben, die eine Durchblutung aufweisen, ist stoffwechselbedingt immer eine gewisse Menge CO₂ im Blut gelöst, wobei sich gezeigt hat, daß das Löslichkeitsprodukt von CO₂ im humanen Vollblut dergestalt ist, daß oberhalb von Temperaturen von 55 °C eine intermediäre Ausgasung erfolgt, die aber bei Unterschreiten dieser Temperatur, also tiefer im Gewebevolumen, reversibel ist. Diese Ausgasung von CO₂ im Blut kann in einfacher Weise durch das zur ursprünglichen Positionierung der Führungsnadel benutzte Ultraschallsystem als Kontraständerung nachgewiesen werden, so daß dadurch in einfacher Weise die 55 °C-Front der Temperaturfeldausbreitung in Echtzeit verfolgt werden kann.
2. Weiterhin hat sich gezeigt, daß auch bei der verwendeten Streuflüssigkeit, einer viskosen Wasser-Glyzerin-Öl-Mischung oder Wasser-Methyzell-Mischung, bei einer Temperatur von etwa 95 °C Wasserdampf gebildet wird, so daß die dadurch entstehenden Gasblasen im Ultraschallbild im Flüssigkeitsdepot leicht beobachtet werden können und die Laserleistung entsprechend zurückgeregelt werden kann, um nicht den Siedepunkt zu überschreiten. Der Wasserdampf geht dann bei Unterschreiten des Löslichkeitsproduktes unmittelbar wieder in Lösung und stellt keine Emboliegefahr dar.

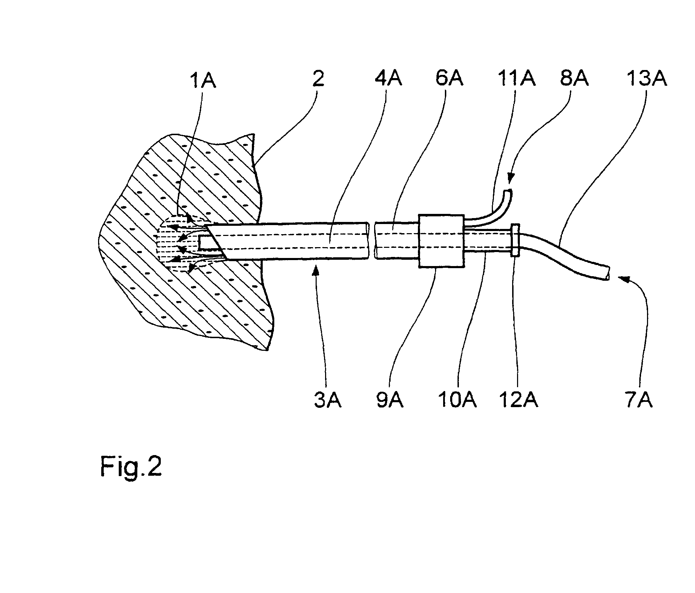

In einer in Fig. 2 schematisch gezeigten Weiterbildung ist eine in Körpergewebe 2 eingeführte Kanüle 3A doppellumig in Form eines integrierten Flüssigkeitslichtleiters ausgeführt dergestalt, daß sie ein koaxial verlaufendes inneres Lumen 4A aufweist, das konzentrisch von einem äußeren Lumen 6A umgeben ist. Dabei dient das innere Lumen 4A als Flüssigkeitslichtleiter, durch den eine optisch transparente Komponente 7A der Streuflüssigkeit zugeführt wird. Das äußere Lumen 6A führt eine streuende oder höher viskose Komponente 8A des Fluids zum distalen Ende der Kanüle 3A, wo sich aus beiden Komponenten ein Fluiddepot 1A ausbildet.

Das proximale Ende der Kanüle 3A weist ein Verzweigungselement 9A auf, von dem eine Zuleitung 10A zum inneren Lumen 4A und eine Zuleitung 11A zum äußeren Lumen 6A abgehen.

Das innere Lumen dient in der Phase der Punktion als Führungsschaft für die Sondiernadel zur Positionierung der Kanüle im zu therapierenden Bereich. Die Kanüle 3A wird mittels einer Punktionsnadel positioniert, anschließend wird die Punktionsnadel entfernt und über die Zuleitung 10A und das innere Lumen 4A zunächst die optisch transparente Fluidkomponente appliziert und sodann in einem zweiten Schritt über die Zuleitung 11A und das äußere Lumen 6A das entstehende Flüssigkeitsdepot 1A mit Streumedium angereichert. Gleichzeitig wird am proximalen Ende über eine Quetschdichtung 12A eine Lichtleitfaser 13A an die Flüssigkeitssäule im Inneren des zentralen Lumens 4A dieser Punktionsnadel angekoppelt, welche sodann als Flüssigkeitslichtleiter dient, wenn über die Lichtleitfaser 13A Laserstrahlung eingekoppelt wird.

In einer bevorzugten Ausführungsform wird als transparentes Trägerfluid eine Mischung aus 80 % Glyzerin und 20 % wasser verwendet, welche einen Brechungsindex besitzt, der im wesentlichen identisch ist mit dem Brechungsindex der ankoppelnden, laserlichtführenden Lichtleitfaser. Das lichtstreuende Medium besteht aus einer 2 bis 5-%igen Ölwasser-Emulsion, beispielsweise Intralipid in geeigneter verdünnung. Bei dieser weiterbildung kommt es konstruktionsbedingt überhaupt nicht mehr zu einem Kontakt zwischen Lichtleitfaser und Gewebe mit dem Risiko einer Karbonisation, sondern der Strahlungseintrag in das Gewebe und das entstehende Depot erfolgt a priori nur durch die Flüssigkeit selbst. Eine Durchmischung der beiden Komponenten Streumedium und transparente Trägersubstanz erfolgt bei Beaufschlagung mit Laserstrahlung selbständig durch die auftretenden thermischen Fluktuationen mit der Folge, daß das aus dem Flüssigkeitslichtleiter austretende Laserlicht nicht unmittelbar in eine Zone hoher Streuung eintritt, sondern graduell mit zunehmender Durchstrahlungstiefe im Depot stärker gestreut wird. Dies führt zu einer nochmals verbesserten Streulichteinbringung in das zu therapierende Gewebevolumen.

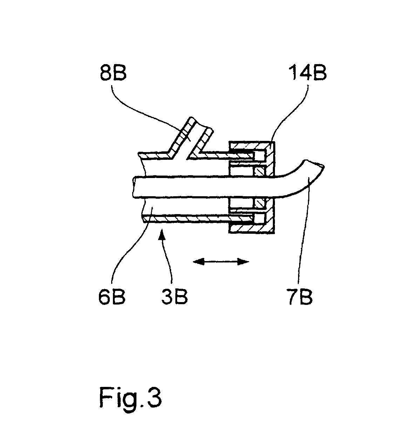

In Fig. 3 ist in einer Detaildarstellung im Längsschnitt der Endabschnitt einer gegenüber der Kanüle 3A nach Fig. 2 modifizierten Kanüle 3B gezeigt, die eine (was in der Figur nicht dargestellt ist) bis zum distalen Ende durchgehende Lichtleitfaser 7B enthält. Ein äußeres Lumen 6B, das über ein seitliches Ansatzstück 8B mit einer (nicht gezeigten) Vorrichtung zur Zufuhr von Streuflüssigkeit in Verbindung steht, ist am proximalen Ende der Kanüle 3B mit einer axial verschiebbaren, flüssigkeitsdichten Verschlußkappe 14B verschlossen. Die Lichtleitfaser 7B ist mit der Verschlußkappe 14B fest verbunden. Über eine Axialverschiebung der Verschlußkappe (oder bei Ausbildung mit Überwurfmutter auch ein Drehen derselben) ist nach dem Einbringen der Kanüle mit vorjustiert endständig mit dem Ende der Kanüle abschließendem Faserende ein Ausfahren des Faserendes aus dem distalen Ende der Kanüle 3B um ca. 1 bis 2 mm in ein dort vorab angelegtes Streufluiddepot hinein möglich.

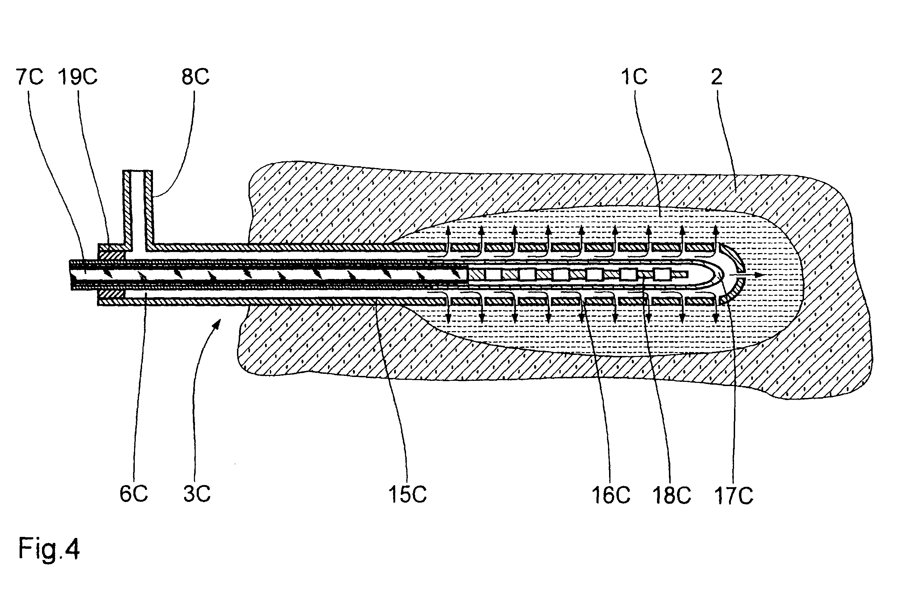

In Fig. 4 ist in einer vereinfachten Längsschnittdarstellung eine weitere Kanüle bzw. ein Katheter 3C zur Erzeugung großvolumiger, langgestreckter Koagulationsnekrosen in Körpergewebe 2 gezeigt. Der Katheter 3C weist einen äußeren Katheterkörper 15C aus transparentem Kunststoff von im wesentlicher zylindrischer Gestalt auf, der an seinem distalen Ende verrundet geschlossen ist und über einen distalen Bereich von einigen cm Länge mit Öffnungen 16C versehen ist. Koaxial zum äußeren Katheterkörper 15C ist in dessen Innenraum 6C eine sich bis nahezu zum distalen Ende des äußeren Katheterkörpers 15C erstreckende, an ihrem distalen Ende ebenfalls verschlossene, innere Glaskanüle 17C angeordnet. Der äußere Katheterkörper weist nahe seinem proximalen Ende, das mit einem Stopfen 19C verschlossen ist, eine seitlich abzweigende Flüssigkeitszuführung 8C auf.

Die Glaskanüle 17C nimmt eine Lichtleitfaser 7C auf, die in ihrem Endbereich von ca. 3 cm Länge mit mehreren ringförmigen, jeweils voneinander äquidistant beabstandeten Mattierungsbereichen 18C versehen ist. Die Lichtleitfaser 7C ist (was in der Figur nicht dargestellt ist) an ihrem proximalen Ende mit einer Laserstrahlungsquelle verbunden.

Durch paraxiale Zuführung von mittel- bis hochviskoser Streuflüssigkeit über die Zuleitung 8C, die über den Katheter-Innenraum 6C und die Öffnungen 16C in das umliegende Gewebe geleitet wird, wird ein den Katheter-Endbereich umgebendes, langgestrecktes Flüssigkeitsdepot 1C gebildet. Bei anschließender Zufuhr von Laserstrahlung wird diese an den mattierten Ringen sowie an der Stirnfläche des Faser 7C sequentiell bzw. periodisch ausgekoppelt.

In Verbindung mit dem randständigen Streufluidepot wird es erstmalig und überraschenderweise möglich, ohne ein erhebliches Fluidvolumen des biokompatiblen Streulichtfluids eine nahezu gleichmäßige verteilung der Laserstrahlung über eine längere zylinderförmige Strecke im Gewebe zu erreichen. Die Verlängerung der Streulichtapplikation, die damit erfindungsgemäß erstmalig möglich wird, erlaubt gleichzeitig höhere Laserleistungen zu applizieren, so daß beispielsweise für die oben zitierte Behandlung eines Prostatalappens mit bis zu 5 cm Längsausdehnung und einem Durchmesser von 2 cm bei Applikation von 15 bis 20 W Laserlichtleistung Strahlungszeiten von lediglich 10 min zur Erzielung des gewünschten Therapieergebnisses notwendig sind. Die Kombination von paraxial zugeführtem Streufluid und in Katheterlängsrichtung verteilt eingekoppelter Laserstrahlung bei der Behandlung sorgt dafür, daß die zunächst im Grundsatz periodisch inhomogen aus der Faser herausgestreute Strahlung zum umliegenden Gewebe hin homogenisiert wird, und es damit zu einem kalkulierbaren und gleichmäßigen Behandlungsergebnis kommt.

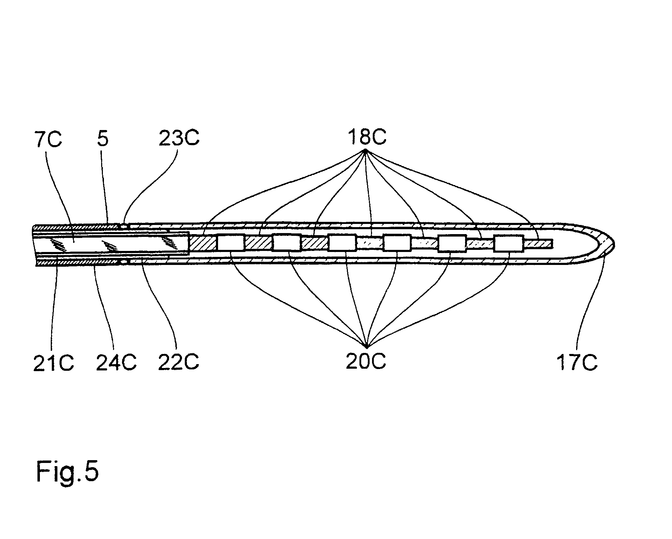

In Fig. 5 ist die innere Kanüle 17C mit der Lichtleitfaser 7C nach Fig. 4 in vergrößerter Darstellung genauer gezeigt. Es ist zu erkennen, wie sich im von Endbereich der dort von ihrem Cladding 21C und Coating 22C befreiten Faser mit einem Längenverhältnis von jeweils 1:1 mattierte Ringe 180 und unbehandelte Faserabschnitte 20C abwechseln. Die mattierten Ringe 18C weisen einen zum Ende der Faser hin abnehmenden Durchmesser bzw. eine zunehmende Rauhtiefe auf. Die Glaskanüle 17C, die klar oder mattiert ausgeführt sein und alternativ auch aus Kunststoff (etwa Polykarbonat) bestehen kann, ist proximal des Mattierungsbereiches durch eine Verklebung 23C mit dem Coating 22C der Faser und einem diese ummantelnden MRI-Marker 24C verklebt.

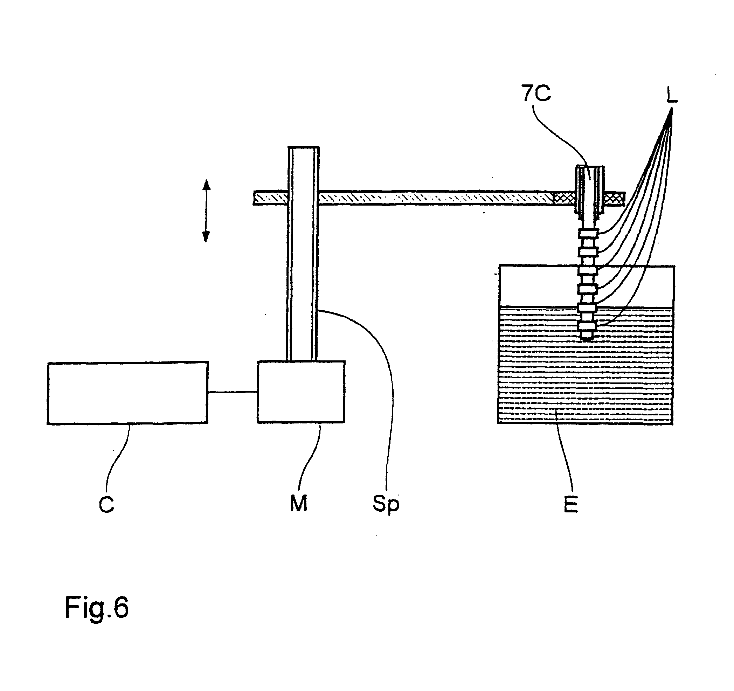

In Fig. 6 ist eine Möglichkeit zur Erzeugung der RingStruktur im distalen Endbereich der Lichtleitfaser 7C skizziert: Das von Coating und Cladding befreite Ende der Basis-Faser wird mit Ringen L aus einem säurefesten Lack (Ätzlack) beschichtet und die Faser unter Einsatz einer Steuerung C, eines Motors M und einer Spindel-Halterung Sp in Faserlängsrichtung stufenweise in eine Ätzflüssigkeit E eingetaucht, wo in den nicht beschichteten Bereichen die Oberfläche der Faser angeätzt wird. Da die Verweildauer in der Ätzflüssigkeit mit zunehmendem Abstand der geätzten Bereiche vom Faserende abnimmt, nimmt auch die Ätztiefe in dieser Richtung ab, wodurch der gewünschte Verlauf der Rauhtiefe bzw. des effektiven Faserdurchmessers erreicht wird. Nach dem Ätzen werden die Lackringe entfernt.

## Patentansprüche

1. Vorrichtung zur thermischen Verödung biologischen Gewebes (2) mittels Laserstrahlung bestehend aus einer mit einer Laserstrahlungsquelle verbindbaren Lichtwellenleiteranordnung, einer doppellumigen Kanüle (3A), deren inneres Lumen (4A) zur Lichtwellenleitung dient, und einer mit dem äußeren und mit dem inneren Lumen der Kanüle (3A) verbundenen Vorrichtung (11A) zur Zuführung einer stark lichtstreuenden, höher viskosen Komponente (8A) und einer optisch transparenten Komponente (7A) einer Streuflüssigkeit, wobei das äußere und das innere Lumen distal offen sind, die Kanüle (3A) aus einem nicht-metallischen, biokompatiblen Material besteht, welches die Laserstrahlung im wesentlichen nicht absorbiert, und konzentrisch doppellumig ausgeführt ist, wobei das innere Lumen (4A) zur Injektion der optisch transparenten Komponente (7A) und das äußere Lumen (6A) zur Zuführung der stark lichtstreuenden Komponente (8A) der Streuflüssigkeit in das Gewebe dient, so daß die optisch transparente Komponente (7A) durch die stark lichtstreuende Komponente (8A) umhüllt wird und Mittel zum koaxialen Einführen oder Ankoppeln eines faseroptischen Lichtwellenleiters (13A) in bzw. an das innere Lumen (4A) vorgesehen sind.

2. Vorrichtung nach Anspruch 1, **dadurch gekennzeichnet, daß** die Kanüle (3) aus im wesentlichen zwei Baugruppen besteht, deren eine die über eine Sondiernadel (S) einzubringende Punktionskanüle selbst ist und eine zweite ein an eben dieser Punktionskanüle nach Entfernung der Sondiernadel anzubringendes fluiddichtes Y-Stück (9A) darstellt, über das einerseits die 2-komponenten Flüssigkeit, die das Laserlicht streut, injiziert werden kann und andererseits der das Laserlicht führende Lichtwellenleiter (7B) vorjustiert fixiert und über einen Verschiebemechanismus definiert axial vorgeschoben werden kann.

3. Vorrichtung nach Anspruch 1, **dadurch gekennzeichnet, daß** das innere Lumen (4A) nach Flutung und Injektion der optisch transparenten Komponente der Streuflüssigkeit selbst als Lichtwellenleiter dient und am proximalen Ende Mittel zur koaxialen Ankopplung eines faseroptischen Lichtwellenleiters an die Flüssigkeitssäule der optisch transparenten Komponente (7A) innerhalb des inneren Lumens (4A) vorgesehen sind.

4. Vorrichtung zur thermischen Verödung biologischen Gewebes (2) mittels Laserstrahlung bestehend aus einer mit einer Laserstrahlungsquelle verbindbaren Lichtwellenleiteranordnung (7C, 17C), bestehend aus einem Lichtwellenleiter (7C) und einem diesen umgebenden inneren Hüllrohr (17C), dergestalt, daß koaxial zu dieser Lichtwellenleiteranordnung (7C, 17C) ein die Laserstrahlung streuendes äußeres Hüllrohr (15C) vorgesehen ist, das aus einem nicht-metallischen, biokompatiblen Material besteht, welches die Laserstrahlung im wesentlichen nicht absorbiert, und die Lichtwellenleiteranordnung (7C, 17C) konzentrisch führt, wobei die Lichtwellenleiteranordnung (7C, 17C) zum Führen des Laserlichtes dient, **dadurch gekennzeichnet daß** das äußere Hüllrohr (15C) zur Zuführung einer stark lichtstreuenden, höher viskosen Komponente (8A) in das Gewebe (2) distale Öffnungen und zusätzliche seitliche Öffnungen (16C) aufweist, durch die die stark lichtstreuende Komponente (8A) aus einem Innenraum (6C) des äußeren Hüllrohres (15C) in einen großvolumigen Bereich des Gewebes (2) zugeführt werden kann, und die Lichtwellenleiteranordnung (7C, 17C) durch die stark lichtstreuende Komponente (8A) umhüllt wird.

5. Vorrichtung nach Anspruch 4, **dadurch gekennzeichnet, daß** die Lichtwellenleiteranordnung eine Lichtleitfaser (7C) aufweist, deren Oberfläche in einem distalen Endabschnitt mehrere in Längsrichtung aufeinanderfolgende mattierte, vorzugsweise ringförmig ausgebildete, Bereiche (18C) aufweist, und daß der Endabschnitt mit einer für die Laserstrahlung transparenten Schutzhülle (17C) versehen ist.

6. Vorrichtung nach Anspruch 5, **dadurch gekennzeichnet, daß** die Schutzhülle (17C) aus einem endständig verschlossenen und am mechanisch festen Teil des Lichtwellenleiters (7C) oder der Kanüle (3C) befestigten Präzisionsglas- oder Hartkunststoffkapillare besteht.

7. Vorrichtung nach Anspruch 5 oder 6, **dadurch gekennzeichnet, daß** die mattierten Bereiche (18C) eine zum distalen Ende der Lichtleitfaser hin zunehmende Rauhtiefe und/oder abnehmenden Durchmesser aufweisen.

8. Vorrichtung nach einem der vorangegangenen Ansprüche, **dadurch gekennzeichnet, daß** die Streuflüssigkeit zur Bildung des Fluiddepots (1A) Öl, Wasser und Glyzerin aufweist, wobei abhängig vom zu verödenden Gewebe (2) und den gewünschten Streueigenschaften 0,1 bis 2 % Öl, 10 bis 50 % Wasser und 50 bis 80 % Glyzerin enthalten sind.

9. Vorrichtung nach einem der vorangegangenen Ansprüche, **dadurch gekennzeichnet, daß** die Streuflüssigkeit zur Bildung des Fluiddepots (1A) eine Mischung von Methylzellulose und Wasser aufweist, wobei in Abhängigkeit von der geforderten Viskosität und den Streueigenschaften 1 bis 30 % Wasser und 70 bis 99 % Methylzellulose enthalten sind.

10. Vorrichtung nach einem der vorangehenden Ansprüche, **dadurch gekennzeichnet, daß** die transparente Komponente (7A) Glyzerin und Wasser, vorzugsweise in einem Mischungsverhältnis von 80:20, und die stark streuende Komponente (8A) Öl und Wasser, vorzugsweise mit einem Anteil von 1 bis 5 % Öl, aufweist.

11. Vorrichtung nach einem der vorangehenden Ansprüche, **dadurch gekennzeichnet, daß** die Streuflüssigkeit Hyalaronsäure und/oder Intralipid aufweist.

12. Vorrichtung nach einem der vorangehenden Ansprüche, **dadurch gekennzeichnet, daß** Mittel zur Kontrolle des Verlaufs der Temperaturfeldausbreitung im Therapieareal hinzugefügt werden, wobei diese Mittel ein Sonographiegerät beinhalten.

13. Vorrichtung nach einem der vorangehenden Ansprüche, außerdem **dadurch gekennzeichnet, daß** der Streuflüssigkeit ein Röntgenkontrastmittel beigefügt ist, um eine röntgenologische Beobachtung zu erlauben.

## Claims

1. Device for thermally obliterating biological tissue (2) by means of laser radiation, consisting of an optical waveguide arrangement which can be connected to a laser radiation source, of a double-lumen cannula (3A) whose inner lumen (4A) serves as optical waveguide, and of a device (11A) which is connected to the outer lumen and to the inner lumen of the cannula (3A) and delivers a strongly light-scattering, highly viscous component (8A) and an optically transparent component (7A) of a scattering liquid, the outer lumen and the inner lumen being open at the distal end, the cannula (3A) being made of a non-metallic, biocompatible material, which does not substantially absorb the laser radiation, and having a concentric double-lumen configuration, the inner lumen (4A) being used to inject the optically transparent component (7A) and the outer lumen (6A) being used to deliver the strongly light-scattering component (8A) of the scattering liquid into the tissue so that the optically transparent component (7A) is surrounded by the strongly light-scattering component (8A), and means are provided for coaxial introduction or coupling of a fibre-optic waveguide (13A) into or onto the inner lumen (4A).

2. Device according to Claim 1, **characterized in that** the cannula (3) is made up of essentially two structural groups, one of which is the puncture cannula itself which is to be introduced via a probe needle (S), and the second of which is a liquid-tight Y-piece (9A) which is to be arranged on this puncture cannula after removal of the probe needle and via which, on the one hand, the two-component liquid which scatters the laser light can be injected, and, on the other hand, the optical waveguide (7B) guiding the laser light can be fixed in a pre-adjusted position and can be advanced axially in a defined manner by means of a displacement mechanism.

3. Device according to Claim 1, **characterized in that** after flooding and injection of the optically transparent component of the scattering liquid, the inner lumen (4A) itself serves as optical waveguide, and means are provided at the proximal end for coaxial coupling of a fibre-optic waveguide to the liquid column of the optically transparent component (7A) inside the inner lumen (4A).

4. Device for thermally obliterating biological tissue (2) by means of laser radiation, consisting of an optical waveguide arrangement (7C, 17C) which can be connected to a laser radiation source and consists of an optical waveguide (7C) and of an inner jacket tube (17C) surrounding the latter, in a configuration which is such that an outer jacket tube (15C) scattering the laser radiation is provided coaxially with respect to this optical waveguide arrangement (7C, 17C), which outer jacket tube (15C) is made of a non-metallic, biocompatible material which does not substantially absorb the laser radiation, and guides the optical waveguide arrangement (7C, 17C) concentrically, the optical waveguide arrangement (7C, 17C) serving for guiding the laser light, **characterized in that** the outer jacket tube (15C) for delivering a strongly light-scattering, highly viscous component (8A) into the tissue (2) has distal openings and additional side openings (16C) through which the strongly light-scattering component (8A) can be delivered from an internal space (6C) of the outer jacket tube (15C) into a large-volume area of the tissue (2), and the optical waveguide arrangement (7C, 17C) is surrounded by the strongly light-scattering component (8A).

5. Device according to Claim 4, **characterized in that** the optical waveguide arrangement has an optical fibre (7C) whose surface, in a distal end portion, has several matted, preferably ring-shaped areas (18C) succeeding one another in the longitudinal direction, and **in that** the end portion is provided with a protective sleeve (17C) transparent for the laser radiation.

6. Device according to Claim 5, **characterized in that** the protective sleeve (17C) consists of a precision glass or hard plastic capillary closed at one end and secured on the mechanically fixed part of the optical waveguide (7C) or of the cannula (3C).

7. Device according to Claim 5 or 6, **characterized in that** the matted areas (18C) have a peak-to-valley height increasing towards the distal end of the optical fibre and/or a diameter decreasing towards the distal end of the optical fibre.

8. Device according to one of the preceding claims, **characterized in that** the scattering liquid for forming the fluid depot (1A) has oil, water and glycerin, and, depending on the tissue (2) to be obliterated and on the desired scattering properties, contains 0.1 to 2 % oil, 10 to 50 % water, and 50 to 80 % glycerin.

9. Device according to one of the preceding claims, **characterized in that** the scattering liquid for forming the fluid depot (1A) has a mixture of methyl cellulose and water, containing 1 to 30 % water and 70 to 99 % methyl cellulose depending on the required viscosity and on the scattering properties.

10. Device according to one of the preceding claims, **characterized in that** the transparent component (7A) has glycerin and water, preferably in a mixing ratio of 80:20, and the strongly scattering component (8A) has oil and water, preferably with a proportion of 1 to 5 % oil.

11. Device according to one of the preceding claims, **characterized in that** the scattering liquid has hyaluronic acid and/or Intralipid.

12. Device according to one of the preceding claims, **characterized in that** means for monitoring the pattern of the temperature field propagation in the treatment area are added, these means including a sonography unit.

13. Device according to one of the preceding claims, additionally **characterized in that** the scattering liquid has an X-ray contrast medium added to it in order to permit radiological observation.

## Revendications

1. Appareillage pour l'oblitération thermique d'un tissu biologique (2) à l'aide d'un rayonnement laser, constitué d'un dispositif de guide d'ondes lumineuses pouvant être raccordé à une source de rayonnement laser, d'une canule à double lumière (3A), dont la lumière interne (4A) sert à faire passer des ondes lumineuses, et d'un appareillage (11A), relié à la lumière externe et à la lumière interne de la canule (3A), destiné à amener un composant (8A), ayant une grande viscosité et provoquant une forte diffusion de la lumière, et d'un composant optiquement transparent (7A) d'un liquide de diffusion, la lumière externe et la lumière interne étant ouvertes en position distale, la canule (3A) étant constituée d'un matériau biocompatible non métallique, qui pour l'essentiel n'absorbe pas le rayonnement laser et qui est constitué de façon à avoir de deux lumières concentriques, la lumière interne (4A) servant à injecter le composant optiquement transparent et la lumière externe (6A) servant à amener dans le tissu le composant (8A), assurant une forte diffusion de la lumière, du liquide de diffusion, de façon que le composant optiquement transparent (7A) soit entouré du composant (8A) assurant une forte diffusion de la lumière, des moyens étant prévus pour l'introduction ou le couplage coaxial d'un guide d'ondes lumineuses à base de fibres optiques (13A) dans ou sur la lumière interne (4A).

2. Appareillage selon la revendication 1, **caractérisé en ce que** la canule (3) est pour l'essentiel constituée de deux sous-ensembles, dont l'un est la canule de ponction proprement dite, destinée à être introduite par l'intermédiaire d'une aiguille de sonde (S), et dont une deuxième représente un raccord en Y (9A), étanche aux fluides, devant être rapporté lui aussi à cette canule de ponction après enlèvement de l'aiguille de sonde, raccord par l'intermédiaire duquel d'une part le liquide à deux composants qui diffuse la lumière laser peut être injecté, et d'autre part le guide d'ondes lumineuses (7B) faisant passer la lumière laser peut être fixé après ajustement préalable, et être poussé vers l'avant dans une direction axiale et parfaitement définie à l'aide d'un mécanisme de poussée.

3. Appareillage selon la revendication 1, **caractérisé en ce que** la lumière interne (4A), après noyage et injection du composant optiquement transparent du liquide de diffusion, sert lui-même de guide d'ondes lumineuses, des moyens étant prévus, au niveau de l'extrémité proximale, pour le couplage coaxial d'un guide d'ondes lumineuses à fibres optiques à la colonne de liquide du composant optiquement transparent (7A) l'intérieur de la lumière interne (4A).

4. Appareillage pour l'oblitération thermique d'un tissu biologique (2) à l'aide d'un rayonnement laser, constitué d'un dispositif de guide d'ondes lumineuses (7C, 17C) pouvant être raccordé à une source de rayonnement lumineux, constitué d'un guide d'ondes lumineuses (7C) et d'un tube de gainage intérieur (17C), entourant ce guide d'ondes, de telle façon que, coaxialement à ce dispositif de guide d'ondes lumineuses (7C, 17C) soit prévu un tube de gainage externe (15C) diffusant le rayonnement laser, tube qui est constitué d'un matériau biocompatible non métallique qui pour l'essentiel n'absorbe pas le rayonnement laser, et qui guide d'une manière concentrique le dispositif de guide d'ondes lumineuses (7C, 17C), le dispositif de guide d'ondes lumineuses (7C, 17C) servant à faire passer la lumière laser, **caractérisé en ce que** le tube de gainage externe (15C) comporte, pour amener un composant (8A) ayant une grande viscosité et assurant une forte diffusion de la lumière dans le tissu (2), des ouverture distales, et des ouvertures latérales additionnelles (16C), par lesquelles le composant (8A), assurant une forte diffusion de la lumière, peut être amené de l'espace intérieur (6C) du tube de gainage externe (15C) dans une zone à grand volume du tissu (2), le dispositif de guide d'ondes lumineuses (7C, 17C) étant alors entouré par le composant (8A) assurant une forte diffusion de la lumière.

5. Appareillage selon la revendication 4, **caractérisé en ce que** le dispositif de guide d'ondes lumineuses comporte une fibre optique (7C), dont la surface comporte, dans un tronçon terminal distal, plusieurs zones (18C), matées, successives dans la direction longitudinale, ayant de préférence une configuration annulaire, et **en ce que** le tronçon terminal est pourvu d'une gaine de protection (17C), transparente au rayonnement laser.

6. Appareillage selon la revendication 5, **caractérisé en ce que** la gaine de protection (17C) est constituée d'un capillaire en verre de précision ou en un plastique rigide, fermé en son extrémité, et fixé à la partie, mécaniquement fixe, du guide d'ondes lumineuses (7C) ou de la canule (3C).

7. Appareillage selon la revendication 5 ou 6,
**caractérisé en ce que** les zones matées (18C) ont une profondeur de rugosité qui augmente, et/ou un diamètre qui diminue vers l'extrémité distale de la fibre optique.

8. Appareillage selon l'une des revendications précédentes, **caractérisé en ce que** le liquide de diffusion comporte, pour former une réserve de fluide (1A), de l'huile, de l'eau ou de la glycérine, auquel cas, indépendamment du tissu (2) à oblitérer et des propriétés souhaitées de diffusion, il contient de 0,1 à 2 % d'huile, de 10 à 50 % d'eau et de 50 à 80 % de glycérine.

9. Appareillage selon l'une des revendications précédentes, **caractérisé en ce que** le liquide de diffusion, pour former la réserve de fluide (1A), comprend un mélange de méthylcellulose et d'eau, qui, en fonction de la viscosité exigée et des propriétés de diffusion, contient de 1 à 30 % d'eau et de 70 à 99 % de méthylcellulose.

10. Appareillage selon l'une des revendications précédentes, **caractérisé en ce que** le composant transparent (7A) est constitué de glycérine et d'eau, de préférence selon un rapport de mélange de 80:20, et le composant (8A) à forte diffusion est constitué d'huile et d'eau, de préférence contenant 1 à 5 % d'huile.

11. Appareillage selon l'une des revendications précédentes, **caractérisé en ce que** le liquide de diffusion comprend de l'acide hyaluronique et/ou de l'Intralipid.

12. Appareillage selon l'une des revendications précédentes, **caractérisé en ce que** des moyens sont ajoutés pour ajuster l'évolution de la propagation du champ de température dans la zone thérapeutique, ces moyens comportant un appareil de sonographie.

13. Appareillage selon l'une des revendications précédentes, **caractérisé par** ailleurs en ce que le liquide de diffusion se voit adjoindre un agent de contraste radiographique, pour permettre une observation aux rayons X.
